# EUROPEAN PATENT SPECIFICATION

(11) **EP 4 039 673 B1**
(45) Date of publication and mention of the grant of the patent: **18.12.2024**
(21) Application number: 20871963.3
(22) Date of filing: 29.09.2020
(51) Int. Cl.: C07C 67/56, C07C 69/22, C07C 69/63, C07C 67/26

(54) **METHOD FOR PRODUCING PROPIONIC ACID DERIVATIVE**
VERFAHREN ZUR HERSTELLUNG EINES PROPIONSÄUREDERIVATS
PROCÉDÉ DE PRODUCTION D'UN DÉRIVÉ DE L'ACIDE PROPIONIQUE

(30) Priority: 30.09.2019 JP 2019179617; 06.01.2020 JP 2020000399
(43) Date of publication of application: 10.08.2022
(73) Proprietor: Daikin Industries, Ltd., Osaka-shi, Osaka 530-0001 (JP)
(72) Inventor: SHIRAI, Atsushi, Osaka-Shi, Osaka 5300001 (JP); YOSHIYAMA, Asako, Osaka-shi, Osaka 530001 (JP); MATSUURA, Makoto, Osaka-shi, Osaka 5300001 (JP); KUROKI, Yoshichika, Osaka-shi, Osaka 5300001 (JP)
(74) Representative: Hoffmann Eitle
(86) International application number: PCT/JP2020/036909
(87) International publication number: WO 2021/065908

(56) References cited:
- WO-A1-2010/071108
- JP-A- H1 135 507
- JP-A- S60 137 928
- JP-A- S61 130 254
- JP-A- S61 130 254
- JP-A- S61 280 468
- YAMADA SHIGEYUKI ET AL: "Easy access to CF2-containing molecules based on the reaction of 2,2,3,3-tetrafluorooxetane with various nucleophiles", ORGANIC & BIOMOLECULAR CHEMISTRY, vol. 9, no. 15, 2011, pages 5493, XP093086804, ISSN: 1477-0520, DOI: 10.1039/c1ob05545c

## Description

### Technical Field

The present disclosure relates to a method for producing a propionic acid derivative and to a composition comprising the same.

### Background Art

Propionic acid derivatives, such as 2,2-difluoropropionic acid esters, are useful compounds as raw materials of e.g. pharmaceuticals and agricultural chemicals. As a method for producing 2,2-difluoropropionic acid esters, a method for reacting 2,2,3,3-tetrafluorooxetane with alcohols or phenols in the presence of an alkali metal halide is known from JP-A-1986-130254.

S. Yamada et al., Org. Biomol. Chem., 9, 5493-5502 (2011) discloses the synthesis of ethyl-3-bromo-2,2-difluoropropanoate by reacting tetrafluorooxetane with MgBr2 in diethylether.

### Summary of Invention

### Technical Problem

In the method of JP-A-1986-130254, after the reaction, (a) a solvent was distilled off, (b) water was added, and (c) an organic solvent was added to perform liquid separation. However, since the reaction liquid contains a large amount of alkali metal fluorides, liquid separation is difficult, which results in poor productivity.

An object of the present disclosure is to solve the above problems and to provide a method for producing a propionic acid derivative having excellent productivity.

### Solution to Problem

The present invention provides a method for producing a compound of formula (1): 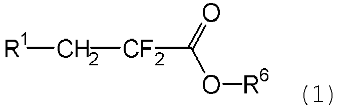 wherein
R¹ is Cl, Br, I or SR, wherein R is H or a hydrocarbon group, and
R⁶ is C₁₋₆-alkyl, haloalkyl or aryl; comprising the steps of
   (A) reacting a compound of formula (2): 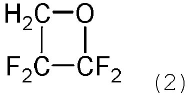 with a compound of the formula M(R¹)ₙ (3), wherein M is cation, n is an integer corresponding to the valence of M, and R¹ is as defined above, and a compound of the formula R⁶-OH (4), wherein R⁶ is as defined above;
   (B) separating, by filtration, a compound of the formula MFₙ (5), wherein M and n are as defined above, from the mixture obtained by the above reaction, and
   (C) performing a liquid separation treatment on a filtrate obtained by the filtration.

The present invention also provides a composition comprising a compound of formula (1) as defined above and a compound of the formula MFₙ (5), wherein M is a cation, and n is an integer corresponding to the valence of M; which composition has a fluorine ion content concentration of > 0 to 150 mg/L.

Preferred embodiments of the invention are as defined in the appended dependent claims and/or in the following detailed description.

### Advantageous Effects of Invention

According to the present disclosure, a method for producing a propionic acid derivative having high productivity is provided.

### Brief Description of Drawings

Fig. 1 is a diagram showing the ¹H-NMR spectrum of ICH₂CF₂COOEt.
Fig. 2 is a diagram showing the ¹⁹F-NMR spectrum of ICH₂CF₂COOEt.

### Description of Embodiments

### 1. Term

The symbols and abbreviations in the present specification can be understood in the sense commonly used in the technical field to which the present disclosure pertains in the context of the present specification, unless otherwise specified, and specifically, , unless otherwise specified, the following applies herein.

The terms "comprise" and "contain" are used with the intention of including the phrases consisting essentially of and consisting of.

The steps, treatments, and operations described herein can be performed at room temperature. "Room temperature" means a temperature of 10-40°C.

The phrase "Cₙ-ₘ" (n and m each represent a number) indicates that the number of carbon atoms is ≥ n and ≤ m, as can typically be understood by a person skilled in the art.

Examples of the "halogen atom" referred to herein include fluorine, chlorine, bromine, and iodine.

Examples of the "substituents" include halogen, cyano, amino, alkoxy and alkylthio. Two or more substituents may be identical to or different from each other. The number of substituents can be 1 to the maximum substitutable number, and it may be 1, 2, 3, or 4.

Examples of "hydrocarbon groups" referred to herein include alkyl, alkenyl, alkynyl, cycloalkyl, cycloalkenyl, cycloalkadienyl, aryl, and aralkyl.

Examples of the "alkyl" referred to herein include linear or branched C₁₋₂₀-alkyl, such as methyl, ethyl, propyl (n-propyl, isopropyl), butyl (n-butyl, isobutyl, sec-butyl, tert-butyl), pentyl, and hexyl.

The "haloalkyl" referred to herein is alkyl substituted with one or more halogen atoms. Examples include fluoromethyl, difluoromethyl, trifluoromethyl (perfluoromethyl), 2-fluoroethyl, 2,2-difluoroethyl, 2,2,2-trifluoroethyl, 1,1,2,2-tetrafluoroethyl, pentafluoroethyl (perfluoroethyl), and linear or branched-chain C₁₋₂₀-haloalkyl, such as groups in which some or all of the fluorine atoms are replaced by other halogen atoms.

Examples of the "alkoxy" referred to herein include linear or branched C₁₋₂₀ alkoxy groups, such as methoxy, ethoxy, propoxy (n-propoxy and isopropoxy), butoxy (n-butoxy, isobutoxy, sec-butoxy, and tert-butoxy), pentyloxy, and hexyloxy.

Examples of the "alkylthio" referred to herein include linear or branched C₁₋₂₀-alkylthio, such as methylthio, ethylthio, propylthio (n-propylthio and isopropylthio), butylthio (n-butylthio, isobutylthio, sec-butylthio, and tert-butylthio), pentylthio, and hexylthio.

Examples of the "alkenyl" referred to herein include linear or branched C₂₋₂₀ alkenyl groups, such as vinyl, 1-propen-1-yl, 2-propen-1-yl, isopropenyl, 2-buten-1-yl, 4-penten-1-yl, and 5-hexen-1-yl.

Examples of the "alkynyl" referred to herein include linear or branched C₂₋₂₀ alkynyl groups, such as ethynyl, 1-propyn-1-yl, 2-propin-1-yl, 4-pentyn-1-yl, and 5-hexyn-1-yl.

Examples of the "cycloalkyl" referred to herein include C₃₋₁₀-cycloalkyl, such as cyclopropyl, cyclobutyl, cyclopentyl, cyclohexyl, and cycloheptyl.

Examples of the "cycloalkenyl" referred to herein include C₃₋₁₀-cycloalkenyl, such as cyclopropenyl, cyclobutenyl, cyclopentenyl, cyclohexenyl, and cycloheptenyl.

Examples of the "cycloalkadienyl" referred to herein include C₄₋₁₀-cycloalkadienyl, such as cyclobutadienyl, cyclopentadienyl, cyclohexadienyl, cycloheptadienyl, cyclooctadienyl, cyclononadienyl, and cyclodecadienyl.

The "aryl" referred to herein the present specification can be monocyclic, bicyclic, tricyclic, or tetracyclic.

The "aryl" referred to herein can be C₆₋₁₈-aryl.

Examples of the "aryl" referred to herein include phenyl, 1-naphthyl, 2-naphthyl, 2-biphenyl, 3-biphenyl, 4-biphenyl, and 2-anthryl.

Examples of the "aralkyl" referred to herein include benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylmethyl, 3-biphenylmethyl, and 4-biphenylmethyl.

### Production Method of Compound of Formula (1)

In one embodiment, the invention relates to a method for producing a compound of formula (1): 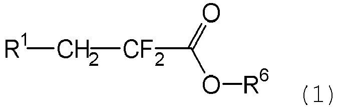 wherein
R¹ is Cl, Br, I or SR, wherein R is H or a hydrocarbon group, and
R⁶ is C₁₋₆-alkyl, haloalkyl or aryl; comprising the steps of
   (A) reacting a compound of formula (2): 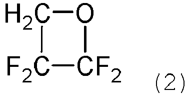 with a compound of the formula M(R¹)ₙ (3), wherein M is cation, n is an integer corresponding to the valence of M, and R¹ is as defined above, and a compound of the formula R⁶-OH (4), wherein R⁶ is as defined above;
   (B) separating, by filtration, a compound of the formula MFₙ (5), wherein M and n are as defined above, from the mixture obtained by the above reaction, and
   (C) performing a liquid separation treatment on a filtrate obtained by the filtration.

### Compound Represented by Formula (1)

R¹ is preferably Cl, Br, I, mercapto or alkylthio; more preferably Cl, Br or I; still more preferably Br or I; and particularly preferably I.

R⁶ is preferably alkyl or haloalkyl; and particularly preferably C₁₋₆-alkyl or C₁₋₆ -haloalkyl.

### Step A

R¹ in formula (3) can be a group corresponding to R¹ in formula (1). The cation M in formula (3) is not particularly limited as long as it is a counter ion of R¹, and examples include hydrogen, metal and ammonium. Examples of the metal include alkali metals and alkaline earth metals. Examples of the alkali metals include lithium, sodium, potassium, and cesium. Examples of the alkaline earth metals include magnesium and calcium. Specific examples of the ammonium include primary to quaternary ammonium. Examples of the primary ammonium include C₁₋₆ alkylamines such as methylamine, ethylamine, propylamine (n-propylamine, isopropylamine), and butylamine, and aniline.

Examples of the secondary ammonium include di-(C₁₋₆-alkyl)amines such as dimethylamine, diethylamine, ethylmethylamine, and dipropylamine, pyrrolidine, imidazole, piperidine, and morpholine.

Examples of the tertiary ammonium include tri-(C₁₋₆-alkyl)amines such as trimethylamine and triethylamine, pyridine, and quinoline.

Examples of the quaternary ammonium include tetra-(C₁₋₆-alkyl)ammonium such as tetramethylammonium and tetraethylammonium.
M is preferably a metal, more preferably an alkali metal or an alkaline earth metal, and even more preferably an alkali metal.

n can be suitably selected according to the valence of M, and is, for example, 1 or 2.

Examples of the compound of formula (3) include NaI, KI, CsI, MgI₂, CaI₂, NaBr, KBr, CsBr, MgBr₂, CaBr₂, NaCl, KCl, CsCl, MgCl₂, and CaCl₂.

The compounds of formula (3) can be used alone or in a combination of two or more.

The lower limit of the amount of the compound of formula (3) can be, for example, 0.1 mol, preferably 0.5 mol, and even more preferably 0.9 mol, relative to 1 mol of the compound of formula (2).

The upper limit of the amount of the compound of formula (3) can be, for example, 10 mole, preferably 5 mole, and even more preferably 3 mole, relative to 1 mol of the compound represented by formula (2).

The amount of the compound of formula (3) can be, for example, 0.1-10 mol, preferably 0.5-5 mol, and even more preferably 0.9-3 mol, relative to 1 mol of the compound of formula (2).

R⁶ in formula (4) can be a group corresponding to R⁶ in formula (1).

Specific examples of the compound of formula (4) include alcohols and phenols. Examples of the alcohols include C₁₋₆-alkanols such as methanol, ethanol, propanol (n-propanol and isopropanol), and butanol. Examples of the phenols include phenol, cresol, and naphthol.

The compound of formula (4) may be used alone or in a combination of two or more.

The lower limit of the amount of the compound of formula (4) can be, for example, 0.1 mol, preferably 0.5 mol, and even more preferably 0.9 mol, relative to 1 mol of the compound of formula (2).

The upper limit of the amount of the compound of formula (4) can be, for example, 10 mol, preferably 5 mol, and even more preferably 3 mol, relative to 1 mol of the compound of formula (2).

The amount of the compound of formula (4) can be, for example, 0.1-10 mol, preferably 0.5-5 mol, and even more preferably 0.9-3 mol, relative to 1 mol of the compound of formula (2).

In the reaction of step A, the compound of formula (4) may be used as a solvent, or a component other than the compound of formula (4) may be used as a solvent. When the compound of formula (4) is used as a solvent, it can be preferably ≥ 10 mol relative to 1 mol of the compound of formula (2).

Examples of the component other than the compound of formula (4) include aliphatic hydrocarbons (e.g., hexane), aromatic hydrocarbons (e.g., toluene, xylene), halogenated hydrocarbons (e.g., dichloromethane, dichloroethane, and chloroform), ethers (e.g., diethyl ether and tetrahydrofuran), ketones (e.g., acetone, methyl ethyl ketone), nitriles (e.g., acetonitrile), esters (e.g., ethyl acetate), amides (e.g., dimethylformamide (DMF) and dimethylacetamide (DMAc)).

The components other than the compound of formula (4) may be used alone or in a combination of two or more.

In the reaction of step A, the reaction temperature and the reaction time are not particularly limited as long as the reaction proceeds.

The lower limit of the reaction temperature can be, for example, -70°C, preferably -20°C, and even more preferably 0°C.

The upper limit of the reaction temperature can be, for example, 150°C, preferably 100°C, and even more preferably 80°C.

The reaction temperature can be, for example, in the range of -70 to 150°C, preferably -20 to 100°C, even more preferably 0-80°C.

The lower limit of the reaction time can be, for example, 0.5 hours, preferably 1 hour, and even more preferably 1.5 hours.

The upper limit of the reaction time can be, for example, 12 hours, preferably 10 hours, and even more preferably 5 hours.

The reaction time is in the range of, for example, 0.5-12 hours, preferably 1-10 hours, and even more preferably 1.5-5 hours.

### Step B

Step B is capable of highly removing the compound of formula (5) from the reaction mixture obtained in step A.

M and n in formula (5) can respectively correspond to M and n in formula (3). Examples of the compound of formula (5) include NaF, KF, CsF, and CaF₂.

The compound of formula (5) can be a compound with low solubility in water and/or an organic solvent. The solubility at 20°C can be, for example, ≤ 100 g/L, preferably ≤ 80 g/L, and even more preferably ≤ 50 g/L.

The method of filtration is not limited. The filtration can usually be performed using a filter material, and preferably using a filter material and a filter aid. The method using a filter material and a filter aid may be pre-coating (a method of filtration using a product in which a filter aid layer is formed on a filter material), or body feeding (a method of filtration by adding a filter aid to the reaction mixture in step A).

Examples of the filter material include paper, metal (e.g., stainless steel), polymer (e.g., cellulose, polypropylene, polyester, and polyamide), glass, ceramics, and cloth.

The filter material is preferably porous, for example, a porous membrane or a porous filter.

The average pore diameter of the filter material is not limited, and is, for example, 0.01-20 pm, preferably 0.01-15 um, and even more preferably 0.01-10 µm.

Examples of the filter aid include diatomite (e.g., Celite (trademark)), filter sand (e.g., manganese sand, manganese zeolite, activated carbon, anthracite, ceramic sand), perlite, and cellulose. The filter aids can be used alone or in a combination of two or more. The filter aid is preferably diatomite.

The filter aid, for example, has an average particle size of 0.5-200 pm, preferably 1-150 pm, and even more preferably 1-100 µm.

The filtration temperature (internal temperature of the reaction mixture subjected to filtration) is not particularly limited. In terms of filtration efficiency, filtration is preferably performed at room temperature or more.

The lower limit of the filtration temperature is preferably 45°C, more preferably 50°C, 55°C, 60°C, or 65°C.

The upper limit of the filtration temperature is preferably 90°C, more preferably 85°C, and even more preferably 80°C.

The filtration temperature is preferably ≥ 45°C, and more preferably 45-90°C.

The filtration can be performed under atmospheric pressure, under pressure, or under reduced pressure, for example, at ≤ 2 MPa, and preferably at ≤ 1 MPa.

### Step C

The present method further includes step (C) of performing a liquid separation treatment on the filtrate obtained by the filtration. By combining the steps B and C, the compound of formula (5) can be further removed.

The liquid separation treatment usually includes the step of adding water and an organic solvent to the filtrate, the step of separating the mixture into the aqueous phase and the organic phase, and collecting the organic phase.

Examples of the organic solvent used in the liquid separation treatment include aliphatic hydrocarbons (e.g., hexane), aromatic hydrocarbons (e.g., toluene and xylene), halogenated hydrocarbons (e.g., dichloromethane, and dichloroethane), ethers (e.g., diethyl ether and tetrahydrofuran), ketones (e.g., methyl ethyl ketone), and esters (e.g., ethyl acetate).

The organic solvents may be used alone or in a combination of two or more. The organic solvent is preferably an ether.

The present method may further include another optional step. Examples of such a step include distillation, concentration, washing, or a combination of two or more steps.

### Composition Comprising Compound of Formula (1) and Compound of Formula (5)

The present composition is a composition comprising a compound of formula (1) and a compound of formula (5), wherein the fluorine ion content concentration is > 0 to 150 mg/L.

The lower limit of the fluorine ion content concentration can be usually the detection limit or 0.001 mg/L.

The fluorine ion content concentration thus can be, for example, 0.001 to 150 mg/L.

The composition further contains the compound of formula (4).

The lower limit of the content of the compound of formula (4) in the composition can be, for example, the detection limit or 0.01 mass%.

The upper limit of the content of the compound of formula (4) in the composition may be, for example, 5 mass%, and preferably 3 mass%.

The content of the compound of formula (4) in the composition can be, for example, ≤ 5 mass%, or 0.01-5 mass%.

### Examples

One embodiment of the present invention is described in more detail by means of the Examples.

### Example 1

A solution of tetrafluorooxetane (75 wt% chloroform solution, 80 g, 0.462 mol) in ethanol (18.8 g) was added dropwise over 1 hour to a suspension of sodium iodide (69.3 g, 0.462 mol) in ethanol (60 g) under ice cooling. After dropwise addition, the temperature was raised to 50°C, and the mixture was stirred under heating for 2 hours. The resulting reaction mixture (internal temperature: 50°C) was filtered using a filter material (paper product, average pore diameter: 4.0 um) and a filter aid (Celite (trademark), average particle size: 12-20 µm), and ethanol was distilled off to obtain ICH₂CF₂COOEt (yield: 90.8%).

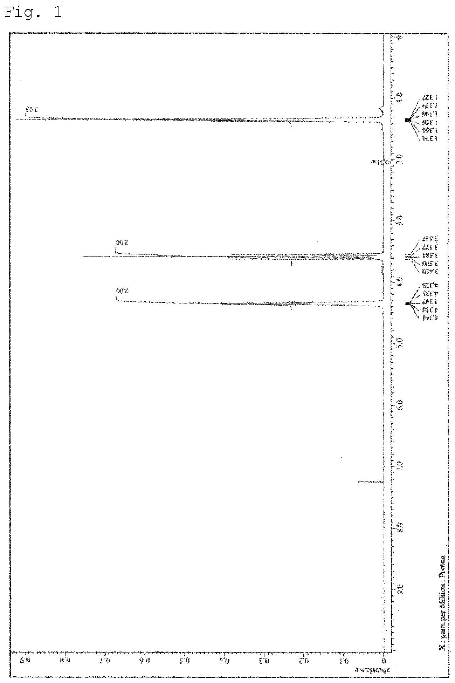

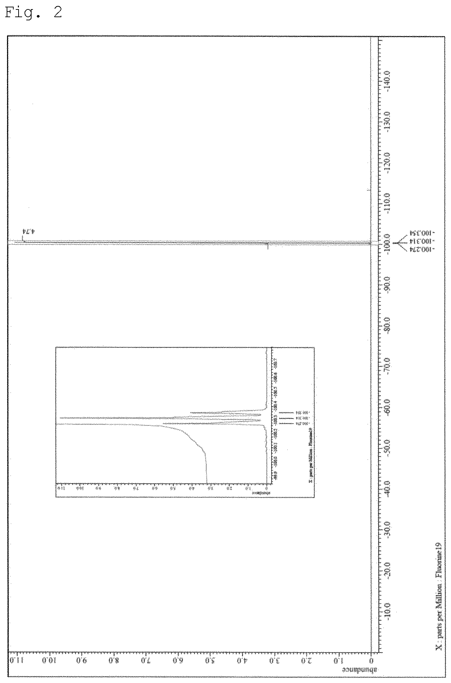

Figs. 1 and 2 show the ¹H-NMR spectrum and ¹⁹F-NMR spectrum of ICH₂CF₂COOEt, respectively.

### Comparative Example 1

ICH₂CF₂COOEt was obtained by the same operation as in Example 1, except that a liquid separation treatment was performed by the addition of water and diethyl ether to the reaction mixture in place of filtration.

### Example 2

ICH₂CF₂COOEt was obtained by the same operation as in Example 1 except that after filtration, water and diethyl ether were added to a filtrate to perform a liquid separation treatment.

The F ion content concentration in the products of Example 1, Comparative Example 1, and Example 2 was measured by the following method.
1. 1 g of a sample was weighed in a plastic container.
2. 1 g of KOH was weighed in a plastic container, and distilled water was added thereto to make 100 g of a KOH aqueous solution.
3. 5 g of the 1% KOH aqueous solution prepared in Item 2 was added to the plastic container of Item 1.
4. The lid of the plastic container of Item 3 was closed, followed by mixing, and the container was allowed to stand. The supernatant was introduced into a disposable syringe with a filter (pore diameter: 0.45 pm), followed by filtration.
5. 1 g of the filtrate obtained in Item 4 was taken out, and 5 mL of distilled water was added thereto, followed by stirring. Thereafter, 4 mL of the upper layer was taken out and transferred into another centrifuge tube. 4 mL of TISAB (total ionic strength adjustment buffer) (produced by Merck Sharp and Dohme) was added thereto, followed by stirring and measurement with an F ion meter.

The following table shows the measurement results of the F ion concentration.

**Table 1**

| | F ion concentration contained in 1 g of sample (mg/L) |
|---|---|
| Example 1 | 104 |
| Comparative Example 1 | 1104 |
| Example 2 | 21 |

### Example 3

ICH₂CF₂COOMe (yield: 81.5%) was obtained by the same operation as in Example 1 except that the ethanol used in Example 1 was changed to methanol.

### Example 4

BrCH₂CF₂COOEt (yield: 61.1%) was obtained by the same operation as in Example 1 except that the sodium iodide used in Example 1 was changed to sodium bromide.

### Examples 5-8

One hundred grams of the reaction mixture obtained in Example 1 was filtered under the conditions shown in Table 2. The time required for the filtration was as shown in Table 2.

**Table 2**

| | Filtration temperature (inner temperature of a reaction mixture) (°C) | Filtration pressure | Filter material ¹⁾ | Filter aid ²⁾ | Time required for filtration |
|---|---|---|---|---|---|
| Example 5 | 65 | Ordinal pressure | Used | Used | 9 min. |
| | | | | | 39 sec. |
| Example 6 | 25 | Ordinal pressure | Used | Used | 13 min. |
| | | | | | 45 sec. |
| Example 7 | 65 | Ordinal pressure | Used | Not used | 4 min. |
| | | | | | 3 sec. |
| Example 8 | 25 | Ordinal pressure | Used | Not used | 12 min. |
| | | | | | 9 sec. |

| | | | | | |
|---|---|---|---|---|---|
| 1) Paper product, average pore diameter: 4.0 µm 2) Celite (trademark), average particle diameter: 12-20 µm | | | | | |

## Claims

1. A method for producing a compound of formula (1): 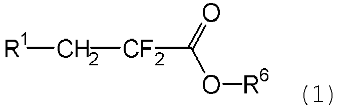 wherein
R¹ is Cl, Br, I or SR, wherein R is H or a hydrocarbon group, and
R⁶ is C₁₋₆-alkyl, haloalkyl or aryl;
comprising the steps of
(A) reacting a compound of formula (2): 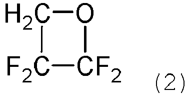 with a compound of the formula M(R¹)ₙ (3), wherein M is cation, n is an integer corresponding to the valence of M, and R¹ is as defined above, and a compound of the formula R⁶-OH (4), wherein R⁶ is as defined above;
(B) separating, by filtration, a compound of the formula MFₙ (5), wherein M and n are as defined above, from the mixture obtained by the above reaction, and
(C) performing a liquid separation treatment on a filtrate obtained by the filtration.

2. The method of claim 1, wherein the filtration is performed at a temperature of ≥ 45°C.

3. The method of claim 1 or 2, wherein the filtration is performed using a filter material and a filter aid.

4. The method of claim 3, wherein the filter aid is at least one of diatomite, filter sand, perlite, and cellulose.

5. The method of claim 3 or 4, wherein the average particle size of the filter aid is 0.5-200 µm.

6. The method of any of claims 1-5, wherein R¹ is Br or I.

7. The method of any of claims 1-6, wherein R⁶ is C₁₋₆-alkyl or haloalkyl, preferably C₁₋₆-alkyl or C₁₋₆-haloalkyl.

8. The method of any of claims 1-7, wherein M is a metal, preferably an alkali metal or an alkaline earth metal, and more preferably an alkali metal.

9. A composition comprising a compound of formula (1): 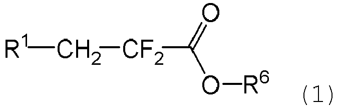 wherein
R¹ is Cl, Br, I or SR, wherein R is H or a hydrocarbon group, and
R⁶ is C₁₋₆-alkyl, haloalkyl or aryl;
and a compound of the formula MFₙ (5), wherein M is a cation, and n is an integer corresponding to the valence of M; which composition has a fluorine ion content concentration of > 0 to 150 mg/L.

10. The composition of claim 9, wherein R¹ is Br or I.

11. The composition of claim 9 or 10, wherein R⁶ is C₁₋₆-alkyl or haloalkyl, preferably C₁₋₆-alkyl or C₁₋₆ haloalkyl.

12. The composition of any of claims 20-30, wherein M is a metal, preferably an alkali metal or an alkaline earth metal, and more preferably an alkali metal.

## Patentansprüche

1. Verfahren zur Herstellung einer Verbindung der Formel (1): 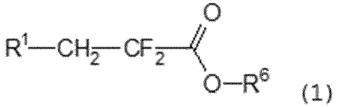 worin
R¹ Cl, Br, I oder SR ist, worin R H oder eine Kohlenwasserstoffgruppe ist, und
R⁶ ein C₁₋₆-Alkyl, Halogenalkyl oder Aryl ist; umfassend die folgenden Schritte:
(A) Umsetzung einer Verbindung der Formel (2): 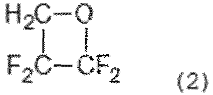 mit einer Verbindung der Formel M(R¹)ₙ (3), worin M ein Kation ist, n eine ganze Zahl ist, die der Wertigkeit von M entspricht, und R¹ wie oben definiert ist, und einer Verbindung der Formel R⁶-OH (4), worin R⁶ wie oben definiert ist;
(B) Abtrennen einer Verbindung der Formel MFₙ (5), worin M und n wie oben definiert sind, aus der durch die obige Umsetzung erhaltenen Mischung durch Filtration, und
(C) Durchführung einer Flüssigkeitsabtrennungsbehandlung an einem durch die Filtration erhaltenen Filtrat.

2. Verfahren gemäß Anspruch 1, wobei die Filtration bei einer Temperatur von ≥ 45°C durchgeführt wird.

3. Verfahren gemäß Anspruch 1 oder 2, wobei die Filtration unter Verwendung eines Filtermaterials und eines Filterhilfsmittels durchgeführt wird.

4. Verfahren gemäß Anspruch 3, wobei das Filterhilfsmittel mindestens eines von Kieselgur, Filtersand, Perlit und Zellulose ist.

5. Verfahren gemäß Anspruch 3 oder 4, wobei die mittlere Teilchengröße des Filterhilfsmittels 0,5-200 µm beträgt.

6. Verfahren gemäß mindestens einem der Ansprüche 1 bis 5, wobei R¹ Br oder I ist.

7. Verfahren gemäß mindestens einem der Ansprüche 1-6, wobei R⁶ ein C₁₋₆-Alkyl oder Halogenalkyl, vorzugsweise C₁₋₆-Alkyl oder C₁₋₆-Halogenalkyl ist.

8. Verfahren gemäß mindestens einem der Ansprüche 1 bis 7, wobei M ein Metall, vorzugsweise ein Alkalimetall oder ein Erdalkalimetall und besonders bevorzugt ein Alkalimetall ist.

9. Zusammensetzung, die eine Verbindung der Formel (1) enthält: 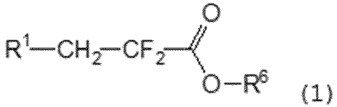 worin
R¹ Cl, Br, I oder SR ist, worin R H oder eine Kohlenwasserstoffgruppe ist, und
R⁶ ein C₁₋₆-Alkyl, Halogenalkyl oder Aryl ist; und eine Verbindung der Formel MFₙ (5), worin M ein Kation ist und n eine ganze Zahl ist, die der Wertigkeit von M entspricht; wobei die Zusammensetzung eine Fluorionenkonzentration von > 0 bis 150 mg/L aufweist.

10. Zusammensetzung gemäß Anspruch 9, wobei R¹ Br oder I ist.

11. Zusammensetzung gemäß Anspruch 9 oder 10, wobei R⁶ ein C₁₋₆-Alkyl oder Halogenalkyl, vorzugsweise C₁₋₆-Alkyl oder C₁₋₆-Halogenalkyl ist.

12. Zusammensetzung gemäß mindestens einem der Ansprüche 20 bis 30, wobei M ein Metall, vorzugsweise ein Alkalimetall oder ein Erdalkalimetall und besonders bevorzugt ein Alkalimetall ist.

## Revendications

1. Procédé de production d'un composé de formule (1) : 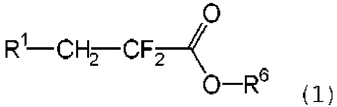 dans lequel
R¹ est un CI, un Br, un I ou un SR, dans lequel R est un H ou un groupe hydrocarboné, et
R⁶ est un alkyle en C₁₋₆, un halogénoalkyle ou un aryle ; comprenant les étapes de
(A) réaction d'un composé de formule (2) : 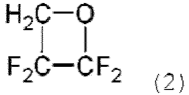 avec un composé de la formule M(R¹)ₙ (3), dans lequel M est un cation, n est un nombre entier correspondant à la valence de M, et R¹ est tel que défini ci-dessus, et un composé de la formule R⁶-OH (4), dans lequel R⁶ est tel que défini ci-dessus ;
(B) séparation, par filtration, d'un composé de la formule MFₙ (5), dans lequel M et n sont tels que définis ci-dessus, du mélange obtenu par la réaction ci-dessus, et
(C) mise en oeuvre d'un traitement de séparation de liquide sur un filtrat obtenu par la filtration.

2. Procédé selon la revendication 1, dans lequel la filtration est mise en oeuvre à une température de ≥ 45 °C.

3. Procédé selon la revendication 1 ou la revendication 2, dans lequel la filtration est mise en oeuvre en utilisant un matériau filtrant et un auxiliaire de filtration.

4. Procédé selon la revendication 3, dans lequel l'auxiliaire de filtration est au moins l'un d'une diatomite, d'un sable filtrant, d'une perlite et d'une cellulose.

5. Procédé selon la revendication 3 ou la revendication 4, dans lequel la taille moyenne de particules de l'auxiliaire de filtration est de 0,5 à 200 µm.

6. Procédé selon l'une quelconque des revendications 1 à 5, dans lequel R¹ est un Br ou un I.

7. Procédé selon l'une quelconque des revendications 1 à 6, dans lequel R⁶ est un alkyle en C₁₋₆ ou un halogénoalkyle, préférablement un alkyle en C₁₋₆ ou un halogénoalkyle en C₁₋₆.

8. Procédé selon l'une quelconque des revendications 1 à 7, dans lequel M est un métal, préférablement un métal alcalin ou un métal alcalino-terreux, et plus préférablement un métal alcalin.

9. Composition comprenant un composé de formule (1) : 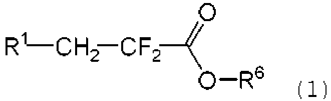 dans laquelle
R¹ est un CI, un Br, un I ou SR, dans laquelle R est un H ou un groupe hydrocarboné, et
R⁶ est un alkyle en C₁₋₆, un halogénoalkyle ou un aryle ;
et un composé de la formule MFₙ (5), dans laquelle M est un cation, et n est un nombre entier correspondant à la valence de M ; laquelle composition présente une concentration en ions fluor de > 0 à 150 mg/l.

10. Composition selon la revendication 9, dans laquelle R¹ est un Br ou un I.

11. Composition selon la revendication 9 ou la revendication 10, dans laquelle R⁶ est un alkyle en C₁₋₆ ou un halogénoalkyle, préférablement un alkyle en C₁₋₆ ou un halogénoalkyle en C₁₋₆.

12. Composition selon l'une quelconque des revendications 20 à 30, dans laquelle M est un métal, préférablement un métal alcalin ou un métal alcalino-terreux, et plus préférablement un métal alcalin.
